# EUROPÄISCHE PATENTSCHRIFT

(11) **EP 4 468 984 B1**
(45) Veröffentlichungstag und Bekanntmachung des Hinweises auf die Patenterteilung: **20.08.2025**
(21) Anmeldenummer: 23773191.4
(22) Anmeldetag: 14.09.2023
(51) Int. Cl.: A61B 18/14, A61B 90/98

(54) **ELEKTROCHIRURGISCHES INSTRUMENT MIT TRANSPONDER, TRANSPONDERKOMMUNIKATIONSSYSTEM UND HERSTELLUNGSVERFAHREN**
ELECTROSURGICAL INSTRUMENT HAVING TRANSPONDER, TRANSPONDER COMMUNICATIONS SYSTEM AND PRODUCTION METHOD
INSTRUMENT ÉLECTROCHIRURGICAL AYANT UN TRANSPONDEUR, SYSTÈME DE COMMUNICATION PAR TRANSPONDEUR ET PROCÉDÉ DE PRODUCTION

(30) Priorität: 23.09.2022 DE 102022124571
(43) Veröffentlichungstag der Anmeldung: 04.12.2024
(73) Patentinhaber: Aesculap AG, 78532 Tuttlingen (DE)
(72) Erfinder: PFISTER, Ralf, 78647 Trossingen (DE); LENZENHUBER, Frederick, 78532 Tuttlingen (DE); BIEBER, Maximilian, 70794 Filderstadt (DE)
(74) Vertreter: Winter, Brandl - Partnerschaft mbB
(86) Internationale Anmeldenummer: PCT/EP2023/075270
(87) Internationale Veröffentlichungsnummer: WO 2024/061726

(56) Entgegenhaltungen:
- WO-A1-2014/017530
- WO-A1-2017/198470
- DE-A1- 102020 116 932
- US-A1- 2014 088 570
- US-A1- 2017 360 505
- US-A1- 2018 071 010
- US-A1- 2018 168 716

## Beschreibung

### Technisches Gebiet

Die vorliegende Offenbarung betrifft ein medizinisches, elektrochirurgisches Instrument, insbesondere HF-Instrument, besonders bevorzugt ein bipolares HF-Instrument, mit einem Transponder, der dafür angepasst ist, elektromagnetische Wellen, insbesondere Datensignale, zu empfangen und/oder zu senden. Daneben betrifft die Erfindung ein medizinisches Transponderkommunikationssystem / Transpondersystem zur Kommunikation bzw. zum Auslesen und/oder Beschreiben eines Transponders sowie ein Herstellungsverfahren, insbesondere Montageverfahren, gemäß den Oberbegriffen der nebengeordneten Ansprüche.

### Hintergrund der vorliegenden Offenbarung

Aus dem Stand der Technik sind Baugruppen mit Transpondern bzw. (medizinische) Markierungselemente mit RFID-Transpondern bzw. RFID-Tags bekannt, welche insbesondere zur Bestückung von chirurgischen Instrumenten herangezogen werden. Mit Hilfe der an den chirurgischen Instrumenten angebrachten RFID-Transpondern ist es möglich, ein solches Instrument zu identifizieren, nachzuverfolgen und zu verwalten. Insbesondere können spezifische Informationen des Instruments ausgelesen werden.

So offenbart beispielsweise die EP 3 193 284 A1 ein medizinisches Markierungselement zur Bestückung chirurgischer Instrumente, welches nachträglich auf ein chirurgisches Instrument oberflächenseitig angebracht werden kann. Dabei weist das Markierungselement eine ringförmige Metallfassung mit einer nichtleitenden Abdeckung auf, in dessen Innenraum ein RFID-Transponder / RFID-Tag eingebracht ist. Eine Außenseite der Metallfassung wird dabei in einer vorbestimmten Lage an dem chirurgischen Instrument, insbesondere durch Schweißen, angebracht.

Die US 2014/0088570 A1 offenbart ein Medizinprodukt, das ein wiederverwendbares oder nicht wiederverwendbares chirurgisches Instrument aus Metall, Polymer oder Kunststoff oder ein Implantat oder Prothesenersatz aus Metall, Polymer oder Kunststoff umfasst, das mit einem Markierungssystem ausgestattet ist. Dieses erlaubt eine schnelle Identifizierung des Instruments. Das Markierungssystem umfasst: eine RFID-Komponente und einen Kunststoffvorsprung, der starr mit dem zu markierenden chirurgischen Instrument oder Implantatersatz verbunden ist.

Die DE 10 2020 116 932A1 offenbart ein medizinisches Instrument mit einem Transpondereinbaumodul, wobei das Transpondereinbaumodul einen Transponder aufweist, der dafür angepasst ist, elektromagnetische Wellen, insbesondere Datensignale, zu empfangen und/oder zu senden. Daneben betrifft die Erfindung ein medizinisches Transponderkommunikationssystem zur Kommunikation bzw. zum Auslesen und/oder Beschreiben eines Transpondereinbaumoduls.

Ferner offenbart die US 2017/0360505 A1 eine elektrochirurgische Gegenelektrode, die für eine Verbindung mit einer Transponder-Erfassungseinheit konfiguriert ist. Die Gegenelektrode umfasst ein leitendes Element mit einer Öffnungsanordnung, die so konfiguriert ist, dass sie den Durchgang eines magnetischen, elektrischen oder elektromagnetischen Abfragesignals von der Transponder-Erfassungseinheit durch das leitende Element und durch die Gegenelektrode ermöglicht. Die Gegenelektrode ist über einer Transponder-Erfassungseinheit positionierbar, so dass die Gegenelektrode auf der Transponder-Erfassungseinheit platziert werden kann und ein Patient auf der Gegenelektrode positioniert werden kann. Die Gegenelektrode ermöglicht die Erfassung eines Transponders, der sich am, im und/oder in der Nähe des Patienten befindet, ohne dass der Patient relativ zur Gegenelektrode neu positioniert werden muss und ohne dass ein zusätzliches Transponder-Lesegerät oder ein Sender über dem Patienten positioniert werden muss.

Insbesondere im Bereich von elektrochirurgischen Hochfrequenz(HF)-Instrumenten, wie etwa bipolaren HF-Instrumenten, weisen diese Instrumente eine beschränkte Lebensdauer hinsichtlich Anwendungszyklen sowie Aufbereitungszyklen auf, in welchen das Instrument einsetzbar und verwendbar ist. Diese Zyklen müssen unbedingt eingehalten werden. Hier bietet sich insbesondere ein Transponder an. Über den Transponder kann eine eindeutige Identifikation des elektrochirurgischen Instruments erfolgen und damit auch eine Erfassung der zugehörigen Anwendungssowie Aufbereitungszyklen. Auf diese Weise kann das Instrument erfasst, protokolliert und nachverfolgt werden, um einen notwendigen Austausch oder Service des individuellen Instruments anzuzeigen. Beispielsweise kann hierbei ein zu einer Instrumenten-ID zugehöriger Zähler inkrementell erhöht werden und gegebenenfalls nach einem Austausch oder Service wieder auf null zurückgesetzt werden.

Ein Nachteil des Standes der Technik ist jedoch, dass Transponder, insbesondere passive Transponder, aufgrund ihrer geringen (signaltechnischen) Übertragungsreichweite in geringem Abstand zu einem Auslesegerät bzw. Lese- und Schreibgerät angeordnet werden müssen. Insbesondere trifft dies bei NFC (Near Field Communication) RFID-Tags zu. Aus diesem Grund ist es bei üblichen Transpondern / RFID-Tags oftmals nur möglich, die RFID-Tags nachträglich auf eine exponierte und gut zu erreichende Außenoberfläche eines medizinischen Instruments aufzubringen, um eine Distanz zwischen Transponder und Auslesegerät minimal zu halten. Dabei ist darauf zu achten, dass die hierfür vorgesehene Außenoberfläche an einer solchen Instrumentenstelle liegt, die nur geringe Nachteile hinsichtlich eines Instrumentenhandlings mit sich bringt und dennoch gleichzeitig eine ausreichende Übertragungsqualität bzw. einen ausreichenden Empfang sicherstellt. Zudem ist im Bereich der elektrochirurgischen Instrumente eine Trennung des RFID-Tags zu den Strom- und Spannungsführenden Komponenten wichtig.

Zudem werden eine Übertragung, ein Empfang bzw. eine Sendeleistung maßgeblich durch die den Transponder umgebenden Bauteile bzw. Strukturen beeinflusst. Dies betrifft auch eine (signaltechnische) Zuverlässigkeit einer Übertragung des Transponders. Insbesondere aber ändert sich eine maximal mögliche Distanz zu einem Lese- und/oder Schreibgerät, so dass je nach Struktur des Instruments und einer Stelle, an dem ein RFID-Markierungselement angebracht ist, ein Auslesen nicht mehr möglich oder zumindest nicht mit hinreichender Zuverlässigkeit möglich ist. Die geometrische Anbausituation eines RFID-Markierungselement und damit eines RFID-Chips des Transponders wirkt sich essentiell auf diese maximale Distanz aus und ist von Instrument zu Instrument verschieden, so dass durch die Markierungselemente nach dem Stand der Technik eine sichere, zuverlässige und vorhersagbare Handhabung nicht gegeben ist.

Daher muss bei einer Entwicklung eines Designs eines medizinischen Instruments ein RFID-Markierungselement sowie eine Umgebung eines RFID-Tags stets berücksichtigt werden und Eingang in das Design finden, da nur so letztlich sichergestellt ist, dass eine entsprechend geeignete umgebende Struktur für den Transponder vorhanden ist. Damit wird eine vom Transponder unabhängige Entwicklung eines medizinischen Instruments erschwert. Da eine Zulassung medizinischer Produkte langwierig und sehr teuer ist, ergibt sich für bestehende als auch neue medizinische Produkte eine besonders hohe Hürde mit entsprechenden Herausforderung einer Anpassung.

Über die signaltechnischen Probleme hinaus liegen beim Stand der Technik auch folgende Nachteile vor. Obgleich derartige RFID-Markierungselemente vergleichsweise kleine Bauteile sind, bilden die im Stand der Technik nachträglich aufgebrachten Markierungselemente mit RFID-Transpondern zusätzliche Angriffsflächen für Verunreinigungen und sind zudem hinderlich bei einer Handhabung der medizinischen Instrumente. Zusätzlich oberflächenseitig angebrachte RFID-Markierungselemente können ferner Spalte und Ritzen bilden, in denen sich Keime ablagern können. Auch ist aufgrund der Gestaltung der Markierungselemente und Anbringung an ein medizinisches Instrument ein irreversibles Anbringen, insbesondere Schweißen, unumgänglich und erschwert bei einem Defekt oder eines vorgesehenen Wechsels des Transponders einen notwendigen Austausch. Des Weiteren bergen außenseitig angebrachte Markierungselemente aufgrund ihrer scharfen Kanten stets ein Risiko eines Aufreißens eines Operationshandschuhs und des Patienten als auch Anwenderschadens.

Auch sind eine Herstellung und eine Montage eines Markierungselements, insbesondere mittels Schweißen, sehr aufwendig und bei einigen medizinischen Instrumenten teils gar nicht möglich, da keine geeigneten Materialien einer Fläche zum Anschweißen zur Verfügung stehen oder da vorgesehene Anbringungsflächen nicht geeignet sind. Insbesondere müssen die Teile eines Gehäuses bzw. Halterung stets des RFID-Markierungselements aus Metall gefertigt werden, um angebracht und angeschweißt werden zu können, was auswählbare geeignete Materialen eines Designs limitiert. Hinzu kommen Vorgaben aus einer Zulassung von medizinischen Instrumenten, welche insbesondere eine wiederholgenaue Herstellung und Montage des RFID-Markierungselements erfordern. Ebenso schirmen metallische Oberflächen RFID-Markierungselemente ab und verringern die Erreichbarkeit dieser.

### Zusammenfassung der vorliegenden Offenbarung

Es ist daher die Aufgabe der vorliegenden Erfindung die Nachteile aus dem Stand der Technik zu beheben oder zumindest zu vermindern und insbesondere eine Elektrodenvorrichtung, ein medizinisches Instrument mit einem Transponder ein Transponderkommunikationssystem, sowie ein Herstellungsverfahren bereitzustellen, das eine Zuverlässigkeit einer signaltechnischen Kommunikation sicherstellt sowie einen Empfang bzw. eine Empfangsleistung und/oder Sendeleistung eines Transponders, insbesondere hinsichtlich einer Distanz zu einem Lese- und Schreibgerät, verbessert. Darüber hinaus soll eine Reinigbarkeit, eine Sterilisierbarkeit sowie eine gute und sichere Handhabung eines medizinischen Instruments sowie eines Transponders verbessert werden. Ebenfalls soll eine Entwicklung, Herstellung, Montage, Wartung, Reparatur sowie ein Austausch eines elektrochirurgischen Instruments verbessert werden. Im Servicefall kann der Transponder schnell und einfach gewechselt werden.

Mit anderen Worten ist es insbesondere die Aufgabe der Erfindung, ein medizinisches elektrochirurgisches Instrument, insbesondere HF-Instrument bereitzustellen, das eine gute und sichere Handhabung sowohl mit Blick auf die Instrumenteneigenschaft als auch auf die Datenübertragungseigenschaft des Transponders gewährleistet. Eine weitere Aufgabe besteht vorzugsweise in einer guten Reinigbarkeit und/oder Sterilisierbarkeit des medizinischen elektrochirurgischen Instruments.

Die Aufgabe wird hinsichtlich eines gattungsgemäßen (medizinischen) elektrochirurgischen Instruments erfindungsgemäß gelöst durch die Merkmale des Anspruchs 1 und hinsichtlich eines gattungsgemäßen medizinischen Transponderkommunikationssystems erfindungsgemäß gelöst durch die Merkmale des Anspruchs 12 und hinsichtlich eines Herstellungsverfahrens erfindungsgemäß durch die Merkmale des Anspruchs 13.

Ein Grundgedanke der vorliegenden Offenbarung kann also darin gesehen werden, dass bei einem elektrochirurgischen Instrument mit zumindest zwei Elektroden ein weiterer, nichtleitender (also mit einem sehr hohen Widerstand ausgestatteten) Isolationskörper vorgesehen ist, welcher einen Transponder aufgenommen hat und derart mit den Elektroden verbunden ist, dass der Transponder zwischen den Elektroden angeordnet ist. Damit ist der Transponder einerseits über den Isolationskörper zu den Elektroden beabstandet und damit elektrisch isoliert und zum anderen ist der Transponder gegenüber den Elektroden zu einer Umgebung hin zurückversetzt. In zumindest einer Elektrode der beiden Elektroden, insbesondere in beiden Elektroden, ist im Bereich des Transponders eine Durchgangsöffnung integriert, so dass durch die Durchgangsöffnung eine Blendenöffnung zur Umgebung hin erstellt wird. Dies begünstigt eine Lese- und Schreibfähigkeit des Transponders, welcher auf diese Weise nicht in einer Draufsicht auf die zwei Elektroden ausgelesen werden kann, sondern zudem noch aus einer Richtung, in welcher die Elektrode zwischen dem Transponder und einem Transponderlesegerät angeordnet ist.

An insbesondere einem proximalen Bereich der zumindest zwei Elektroden wird also zwischen den Elektroden ein in einem Isolationskörper gelagerter Transponder integriert und zudem durch die Blendenöffnung in zumindest einer Elektrode eine Datenübertragung (Auslesefähigkeit) deutlich verbessert.

Mit anderen Worten wird eine Aufnahme eines Transponders, insbesondere eines RFID-Tags, ganz besonders eines Glass Tags bei HF Instrumenten im (jeweils) in einem proximalen Isolationskörper zwischen den beiden gegenüberliegenden Elektroden vorgeschlagen, vorzugsweise mittig zur Außenkontur des Isolationskörpers. Durch entsprechende geometrische Aussparungen/ Öffnungen in zumindest einer Elektrode, insbesondere beider, der gegenüberliegenden Elektroden im Bereich des Transponders (insbesondere des Glass Tags) kann der Transponder in alle Richtungen bzw. um 360° ausgelesen werden.

Mit noch ganz anderen Worten wird ein elektrochirurgisches Instrument, insbesondere HF-Instrument, (mit einer Elektrodenvorrichtung) bereitgestellt mit einer ersten Elektro und einer zweiten Elektrode, die sich, insbesondere distal des Instruments, gegenüberliegen. Ferner weist das Instrument einen (elektrischen) Isolationskörper/ ein Isolationsmodul auf, der elektrisch isolierend ausgebildet ist, in welchem ein Transponder, bevorzugt ein RFID-Transponder, besonders bevorzugt ein Glass-Transponder, aufgenommen/ eingesetzt ist. Dieser Isolationskörper verbindet die erste und zweite Elektrode miteinander, wobei der in dem Isolationskörper aufgenommene Transponder zwischen der ersten und zweiten Elektrode angeordnet ist, insbesondere symmetrisch und/oder mittig zwischen der ersten und zweiten Elektrode. Ferner ist in der ersten Elektrode und/oder in der zweiten Elektrode im Bereich des Transponders bzw. auf Höhe des Transponders eine geometrische Aussparung/ Öffnung/ Blendenöffnung ausgebildet, so dass die Elektrode eine für elektromagnetische Wellen signalundurchlässige Blende mit einer signaldurchlässigen Blendenöffnung ausbildet.

Der Transponder ist dabei in dem Isolationskörper (elektrisch isolierenden Körper) aufgenommenen und lagefixiert. Während also die Elektrode etwa aus Metall ausgebildet ist, um den elektrischen Strom zu leiten bedingt dieses Material andererseits, dass die Elektrode signalundurchlässig ist. Durch die Schaffung einer speziellen geometrischen Aussparung bzw. Blendenöffnung in der Elektrode wiederum, kann eine signaltechnische Übertragung auch in Richtung der Elektrode, also in einer Verlängerung einer Geraden von dem Transponder zu der Elektrode, hin zu einer Umgebung bereitgestellt werden. Werden nun in den zumindest zwei Elektroden, insbesondere in allen Elektroden, eine solche Blendenöffnung vorgesehen, so ist eine signaltechnische Datenübertragung in 360° um den Transponder herum möglich. Insbesondere wird durch die Blendenöffnung sogar eine mögliche maximale Distanz zu einem Lesegerät vergrößert. Es wird also direkt in der Elektrode eine speziell zu dem Transponder angeordnete Blende mit vordefinierten geometrischen Abmessungen (etwa der Blendenöffnung) ausgebildet, die einen Empfang des Transponders und insbesondere eine mögliche Distanz zu einem externen Lese- und/oder Schreibgerät, verbessert.

Der Transponder lässt sich so noch besser und flexibler in das elektrochirurgische Instrument integrieren.

Der Isolationskörper selbst ist insbesondere signaldurchlässig und hat vorzugsweise keinen oder nur einen geringen Einfluss auf einen (signaltechnischen) Empfang des Transponders bzw. auf eine elektromagnetische Wechselwirkung. Der Isolationskörper kann insbesondere hinsichtlich einer guten Reinigbarkeit und Sterilisierbarkeit ausgelegt werden und ferner hinsichtlich eines Einbaus oder Einsetzens in das medizinische Instrument bzw. der Elektroden in den Isolationskörper.

Ganz anders hierzu verhält sich die Blende bzw. die Elektrode mit der Blendenöffnung, welche ein signalundurchlässiges bzw. ein für elektromagnetische Wellen, zumindest für einen bestimmten Frequenzbereich, nicht durchlässiges Material aufweist oder aus einem solchen besteht. Die Blendenöffnung hat einen essentiellen Einfluss auf eine elektromagnetische Wechselwirkung zwischen dem Transponder und einer Umgebung und ist insbesondere speziell dafür ausgelegt und angepasst, elektromagnetische Signale bzw. elektromagnetische Wellen des Transponders zu bündeln und/oder zu verstärken.

Durch diesen Aufbau ist die Elektrodenvorrichtung des elektrochirurgischen Instruments als Einheit optimal ausgelegt und ein Empfang und eine Übertragung zum bzw. vom Transponder zur Umgebung ist deutlich verbessert. In Folge ist auch eine Kommunikation zwischen eines im Bereich der Oberseite bzw. der Instrumentenkörper-Oberfläche anordbaren (Transponder-)Lese- und/oder Schreibgeräts verbessert, so dass ein sicheres und zuverlässiges Lesen und/oder Schreiben des Transponders durch die Gestaltung der Elektrodenvorrichtung selbst, auch über größere Distanzen, gewährleistet ist.

Der Begriff "im Bereich des Transponders" meint etwa, dass entlang einer Längsachse des Instruments gesehen auf einer ähnlichen Höhe die Blendenöffnung vorgesehen ist, insbesondere in einem Bereich der Elektrode, der dem Transponder geometrisch gesehen am nächsten ist.

Es wird an dieser Stelle hervorgehoben, dass die Elektrodenvorrichtung mit der ersten und zweiten Elektrode sowie dem Isolationskörper mit dem Transponder eine selbstständige Erfindung darstellt und unabhängig beanspruchbar ist bzw. für die gesonderter Schutz beanspruchbar ist und die zum Gegenstand einer separaten Anmeldung gemacht werden soll.

Mithilfe des Instruments mit Transponder kann beispielsweise ein produktbezogener Aufbereitungszyklus mit entsprechenden Informationen erfasst, dokumentiert und daraus gewonnene Erkenntnisse weiterverarbeitet werden. Ein solcher Aufbereitungszyklus kann beispielsweise eine Reinigung und/oder eine Sterilisation und/oder ein Ölen umfassen. Mit Hilfe des Instruments mit Transponder kann ein sogenanntes Tracking sowie ein Lifecycle Management einer medizinischen Vorrichtung/ eines medizinischen Produkts, insbesondere eines medizinischen Instruments, vorgenommen werden. Die Information dient auch dem Hersteller hinsichtlich einer Beweisführung in Reklamationsfällen. Ferner kann ein Nutzer ein mit dem Transponder bestückte medizinische Vorrichtung, insbesondere medizinisches Instrument, erst bei individueller Notwendigkeit einer Instandhaltung dieses einer Instandhaltung unterziehen und muss sich nicht mehr zwingend an ein vordefiniertes Instandhaltungsintervall heranziehen. Dies begünstigt eine Verfügbarkeit und Bereitstellung von medizinischen Instrumente, da hierdurch die Wartungsintervalle individuell verlängert werden können.

Vorteilhafte Ausführungsformen sind in den Unteransprüchen beansprucht und werden insbesondere nachfolgend erläutert.

Gemäß einer Ausführungsform kann die Blendenöffnung der Elektrode länglich oder schlitzförmig oder langlochförmig, insbesondere als Langloch, ausgebildet sein. Insbesondere bei Elektroden, die sich pinzettenartig sich in eine Richtung erstrecken und eine etwa blechförmige oder plattenförmige Grundstruktur aufweisen, kann durch eine Einbringung einer länglichen Aussparung eine Blendenöffnung in der Elektrode selbst integriert werden.

In einer Ausführungsform kann der Isolationskörper als Material einen thermoplastischen Kunststoff, insbesondere Polypropylen (PP) oder Polyethylen (PE), aufweisen, insbesondere aus diesem bestehen, vorzugsweise ein Kunststoffspritzgussteil sein, um eine elektrische Isolation bereitzustellen. Kunststoffe sind gute elektrische Isolatoren und können einfach und kostengünstig hergestellt werden. Zudem sind Materialien wie Polypropylen (PP) und Polyethylen (PE) biokompatibel.

Insbesondere kann der Isolationskörper zumindest zweiteilig ausgebildet sein und einen Hauptkörper mit einer Aufnahme, insbesondere einer Aussparung, für eine Aufnahme des Transponders aufweisen, und ferner einen Verschlusskörper, insbesondere eine Art komplementären Deckel oder Pfropfen aufweisen, der über einen Form- und/oder Kraftschluss mit dem Hauptkörper verbindbar ist und die Aufnahme hermetisch gegenüber einer Umgebung abschließt, so dass der Transponder lagefixiert und verliersicher aufgenommen ist. Die zumindest zweiteilige Ausgestaltung erlaubt eine einfache Herstellung der beiden Körper, ein Einsetzen des Transponders in die (Transponder-)Aufnahme und ein Einfaches verschließen dieser Aufnahme mit eingesetztem Transponder, um diesen verliersicher zu halten und zudem eine Sterilität des üblicherweise unsterilen Transponders gegenüber Umgebung zu gewährleisten.

Insbesondere kann der Isolationskörper steril oder sterilisierbar ausgestaltet sein.

Vorzugsweise kann der Verschlusskörper stoffschlüssig mittels Warmverformen oder Ultraschallschweißen mit dem Hauptkörper verbunden sein. Durch diese Weise kann der Verschlusskörper nicht versehentlich vom Hauptkörper abfallen und zudem ist eine hermetische Versiegelung des Transponders gewährleistet.

Gemäß einer Ausführungsform kann der Isolationskörper zwei, insbesondere symmetrisch zu einer Längsachse oder Symmetrieebene des Isolationskörpers verlaufende, insbesondere parallel verlaufende, Durchgangskanäle /Durchgangsöffnungen, insbesondere Schlitze aufweisen, in welche jeweils die erste Elektrode und die zweite Elektrode einsetzbar bzw. eingesetzt ist. Die Elektroden können jeweils distal und proximal aus dem Isolationskörper vorstehen /herausragen/ hervorstehen und insbesondere über eine Presspassung in dem Isolationskörper gehalten werden. Durch diese Gestaltung des Isolationskörpers ist dieser als Verbindungskörper der beiden Elektroden konfiguriert. Diese Elektroden werden in die Durchgangsöffnungen durchgesteckt und stehen sowohl distal hervor, um ein Gewebe eines Patienten manipulieren zu können und stehen auch proximal aus dem Isolationskörper hervor, um entsprechend elektrisch angeschlossen zu werden. Eine solche Ausgestaltung mit den beiden Durchgangsöffnungen, insbesondere Schlitzen, ist eine effiziente und kostengünstige Lösung.

Gemäß einer weiteren Ausführungsform kann der Isolationskörper in der Durchgangsöffnung, insbesondere dem Schlitz, eine Verjüngung oder einen Rast-Vorsprung, insbesondere einen komplementär zur Blendenöffnung ausgebildeten Rast-Vorsprung, aufweisen, so dass das die zugehörige Elektrode mit der Blendenöffnung mittels elastischem Formschluss über den Rast-Vorsprung (als Verjüngung) elastisch gehalten/fixiert ist. Ist also in der Elektrode die Blendenöffnung integriert und damit gewissermaßen eine Aussparung oder Loch vorhanden, und sitzt die Elektrode (nach Einführung) in der Durchgangsöffnung, wobei die Blendenöffnung im Bereich des Transponders ist, so lässt sich die Geometrie der Blendenöffnung verwenden, um zusammen mit einem komplementären Rast-Vorsprung, eine elastische (und damit auch wieder lösbare Halterung zu erreichen, welche jedoch nur mit einem vordefinierten Kraftaufwand lösbar ist. Der Rast-Vorsprung steht dabei in die Blendenöffnung hinein und ist elastisch vorgespannt. Insbesondere kann eine Rampenstruktur als Rast-Struktur verwendet werden, um eine Montage zu vereinfachen, jedoch eine Demontage zu erschweren. Die Elektrode lässt sich somit relativ gesehen einfach und leicht in die Durchgangsöffnung des Isolationskörpers hineinschieben, wobei die Elektrode über die Rampe hineingleitet, und sobald der rampenförmige Rast-Vorsprung in die Blendenöffnung hineinsteht bildet dieser einen Hinterschnitt, etwa einen senkrechten Hinterschnitt aus, so dass eine Demontage nur mit einem Auflösen des Hinterschnitts erfolgen kann, etwa durch elastisches Zurückziehen des Rast-Vorsprungs aus der Blendenöffnung.

Insbesondere kann der Isolationskörper, insbesondere der Isolationskörper und die erste und zweite Elektrode, symmetrisch zu einer Symmetrieebene ausgebildet sein, so dass auch der Transponder symmetrisch zwischen der ersten und zweiten Elektrode angeordnet ist.

Weiter bevorzugt ist die Blendenöffnung welche als Durchtritt für elektromagnetische Wellen oder Signale, insbesondere Radiosignale, dient, länglich oder schlitzförmig ausgebildet und weist eine (Schlitz-)Breite auf, die dem Produkt von Spulendurchmesser (des Transponders, insbesondere des Glass-Tags) und einem Idealfaktor entspricht, wobei der Idealfaktor im Bereich von 1,3 bis 2,2 liegt, weiter vorzugsweise in einem Bereich von 1,6 bis 1,9 liegt und besonders bevorzugt 1,75 beträgt. In anderen Worten ausgedrückt, weist die Blendenöffnung, die parallel zu dem Transponder, insbesondere dem Glass-Tag, ist, eine Breite auf, die größer als der Spulendurchmesser und/oder eine Breite des Transponders, insbesondere eines Durchmessers des Glass-Tags, ist.

Weiter bevorzugt weist die längliche oder schlitzförmige Blendenöffnung eine Länge auf, die -30% bis zu +50% der Gesamtlänge eines Spulenkerns, insbesondere Ferritkerns, des Transponders beträgt, weiter bevorzugt eine Länge von 0% bis zu +30% der Gesamtlänge des Ferritkerns und besonders bevorzugt eine Länge von +15% der Gesamtlänge des Ferritkerns beträgt. In anderen Worten ausgedrückt, weist die Blendenöffnung, die parallel zu dem Transponder, insbesondere dem Glass-Tag ist, eine Länge auf, die vorzugsweise größer als die Länge des Spulenkerns, insbesondere Ferritkerns, und/oder des Transponders, insbesondere Glass-Tags ist.

Vorzugsweise kann der Isolationskörper, insbesondere die Oberseite, eine Farbe aufweisen oder farblich markiert sein, so dass im Wege einer Farbkodierung dem Isolationskörper und damit der Elektrodenvorrichtung bzw. dem medizinischen Instrument eine Information zugewiesen ist. So kann auch insbesondere ein Instrumentensystem mit jeweils einem passenden Set von medizinischem Instrument und kompatiblen Transponder geschaffen werden.

Gemäß einer Ausführungsform kann der Transponder eine zylindrische Form, insbesondere mit abgerundeten Enden, mit einer Transponder-Längsachse aufweisen, die Blendenöffnung der Blende länglich oder schlitzförmig oder langlochförmig ausgebildet sein und eine Blendenöffnungs-Längsachse aufweisen, und die Transponder-Längsachse parallel zu der Blendenöffnungs-Längsachse, von dieser beabstandet und insbesondere symmetrisch zu dieser angeordnet sein. Damit ist der Transponder symmetrisch zu den Blendenöffnungen der Elektroden angeordnet und kann sehr gut ausgelesen werden.

Vorzugsweise kann ein Abstand zwischen dem Transponder und der Blendenöffnung minimal 2mm und/oder maximal 20mm betragen, insbesondere zwischen einer Längsachse des Transponders und einer Längsachse der Blendenöffnung; und/oder ein kürzester Abstand zwischen der ersten Elektrode und zweiten Elektrode kann minimal 4mm und/oder maximal 40mm betragen.

Insbesondere ist die Elektrode flächig oder blechartig, insbesondere flach / plan, ausgebildet, und weist (im Bereich der Blendenöffnung) eine konstante Höhe (Dicke) senkrecht zur Blendenöffnung auf.

Insbesondere kann die Elektrode gesamt aus Metall hergestellt sein. Vorzugsweise besteht die Elektrode aus Edelstahl. Metall ist für elektromagnetische Wellen undurchlässig. Edelstahl lässt sich besonders gut sterilisieren.

Insbesondere kann in dem Isolationskörper eine zylinderförmige Aufnahme in Form einer Aussparung ausgebildet sein, welche nach proximal oder distal eine Öffnung aufweist, um den Transponder von proximal oder von distal einzusetzen.

Ferner kann insbesondere der Transponder gegenüber der Blendenöffnung beabstandet und zurückversetzt angeordnet sein.

Insbesondere kann die Aufnahme/ Aussparung in dem Isolationskörper passgenau auf einen Glass-Tag als Transponder abgestimmt sein, so dass dieser nicht herausfällt und reibschlüssig gehalten wird. Vorzugsweise kann eine Presspassung des Transponders in der Aufnahme des Isolationskörpers ausgebildet sein, also etwa ein kleinerer Durchmesser der Aufnahme als der Durchmesser eines Glass-Tags. Insbesondere kann der Transponder in der Aufnahme des Isolationskörpers mittels Presspassung aufgenommen sein. Transponder und Isolationskörper sind also derart aufeinander abgestimmt sind, dass der Transponder im eingesetzten Zustand eine Presspassung ausbildet.

Insbesondere kann auch die Elektrode, insbesondere die erste und zweite Elektrode, mit dem Isolationskörper eine Presspassung ausbilden. Elektrode und Isolationskörper sind also derart aufeinander abgestimmt sind, dass die Elektrode im eingesetzten Zustand eine Presspassung ausbildet.

Insbesondere kann der Transponder in den Isolationskörper, insbesondere zudem auch die Elektroden, werkzeuglos zusammensetzbar sein und/oder werkzeuglos in die vorbereitete Aufnahme (bzw. Durchgangsöffnung) einsetzbar und entnehmbar sein. Die Elektrodenvorrichtung ist also werkzeuglos montierbar.

Insbesondere kann die Aufnahme in dem Isolationskörper als zylinderförmige Ausnehmung bzw. Bohrung ausgestaltet sein.

Insbesondere kann eine Breite der Blendenöffnung (also senkrecht zu einer Längsachse der Elektrode) minimal 5mm und/oder maximal 15mm betragen.

Insbesondere kann ein Abstand (von Aufnahme des Transponders und damit) des Transponders zu der Durchgangsöffnung, in welche die Elektrode eingesetzt ist, minimal 2mm und/oder maximal 20mm betragen Insbesondere kann der Isolationskörper als werkzeugfallendes Kunststoffspritzgussteil ausgebildet oder hergestellt sein. Insbesondere liegt dem Isolationskörper die Idee zugrunde, dass wenn insbesondere ein signaldurchlässiges Isolationskörper gewählt wird (aufweisend z.B. Thermoplast, Duroplast, Kunststoffe allgemein und/oder Silikon als Material), die Lese - und/oder Schreibdistanz durch einen den Transponder zumindest teilweise umgebenden Elektrode als Metallschirm/Reflektor/Blende mit Blendenöffnung, welcher den Transponder zu der Oberseite und damit zu einem dort anordbaren Lese- und Schreibvorrichtung beabstandet, mit einer geometrisch definierten Öffnung optimiert.

Gemäß einer weiteren Ausführungsform der Erfindung kann der Isolationskörper und/oder die Elektrode im Bereich der Blendenöffnung mit einem Biozid versehen sein, so dass ein Risiko eine Keimbildung weiter verringert wird.

Vorzugsweise kann der Transponder ein passiver RFID-Transponder sein.

Vorzugsweise ist der Transponder ein RFID-Transponder, besonders bevorzugt ein Glass-Tag, zum Speichern von zu einem bestimmten medizinischen Instrument zugehörigen Informationen. Dieser RFID-Transponder ist dafür angepasst, entsprechend von Anforderungen eines vordefinierbaren Prozesses individualisiert zu werden. Insbesondere weist der RFID-Transponder bzw. RFID-Tag auf:
- einen Mikrochip, vorzugsweise mit einer Abmessung von weniger als 2 Millimeter,
- eine Antenne, vorzugsweise in der Form einer Spule, besonders bevorzugt mit einem innenliegenden stabförmigen Ferritkern, um welchen die Spule gewunden ist, und
- eine Ummantelung, wobei die Ummantelung vorzugsweise wasserdicht und/oder luftdicht ist und bevorzugt die Elektronik des Transponders vor der Umgebung schützt.

Gemäß einer weiteren Ausführungsform kann der Transponder auch ein aktiver RFID-Transponder sein, der wenigstens eine Energiequelle aufweist, vorzugsweise in Form einer Batterie, eines Akkumulators und/oder eines Kondensators.

Vorzugsweise ist der Transponder dafür vorgesehen und angepasst, wenigstens eine der folgenden Informationen verschlüsselt oder unverschlüsselt zu speichern:
- Allgemeiner Zustand,
- Lebensdauer / Lebensdauerende,
- Wartungsintervall,
- Performance und Eignung für Folge-OP,
- Minderpflege von Produkt und ggf. Produktschäden,
- Temperaturüber- und Temperaturunterschreitungen und ggf. Produktschäden,
- Artikelnummer,
- Seriennummer, und/oder
- Kunde.

Dadurch wird es möglich, bei medizinischen Instrumenten, insbesondere in Kombination mit einer individualisierten Lagerung, Aufbereitungszyklen zu erfassen, zu zählen und diese Informationen im medizinischen Instrument selbst zu sichern. Insbesondere kann dadurch erfasst werden, ob denn alle notwendigen Prozessschritte eingehalten und durchgeführt wurden. Eine Anzahl der Aufbereitungszyklen kann insbesondere ein proportionales Maß zu vorstehenden Informationen sein.

Vorzugsweise weist der Transponder eine zylindrische Form mit abgerundeten Enden auf. Die Form des Transponders, insbesondere des Glass-Tags, ist insbesondere eine Pillenform.

Der Isolationskörper mit der Aufnahme für den Transponder kann insbesondere wahlweise frei/offen nach außen verbleiben oder mit einem signaldurchlässigen Werkstoff aufgefüllt/verschlossen sein. Insbesondere kann der Transponder bereits per se nach Art einer Verschlusskappe geformt sein, derart, dass mit Einsetzen des Transponders in den Isolationskörper dieses bzw. deren Öffnung vom Transponder selbst nach außen (wasser-/luftdicht) verschlossen wird.

Vorzugsweise ist der Transponder gegenüber der Blendenöffnung von der Umgebung in Richtung der beiden Elektroden in Verlängerung gesehen, mittig und symmetrisch zueinander angeordnet. Dadurch liegt der Transponder mittig in der Blendenöffnung und ein Empfang wird verbessert.

Gemäß einer bevorzugten Ausführungsform kann die Größe eines Glass-Tags 2mm im Durchmesser und 12mm in der Länge betragen. Alternativ kann die Abmessung bevorzugt auch 3mm im Durchmesser und 13mm in der Länge oder bevorzugt 4mm im Durchmesser und 22mm in der Länge betragen.

Zudem kann der Transponder vorzugsweise ein Frequenzband im Bereich von 12 bis 15 **MHz,** vorteilhafterweise in dem Bereich von 13 bis 14 **MHz,** weiter bevorzugt in dem Bereich von 13,4 bis 13,7 MHz und besonders bevorzugt von 13,56 MHz nutzen.

Die Aufgabe der vorliegenden Offenbarung wird hinsichtlich eines medizinischen Transpondersystems / Transponderkommunikationssystem erfindungsgemäß dadurch gelöst, dass dieses ein offenbarungsgemäßes medizinisches Instrument mit Transponder aufweist sowie eine mit dem Transponder signaltechnisch koppelbare Lese- und/oder Schreibvorrichtung aufweist, die insbesondere mit einer oder selbst als eine Instrumentenhalterung ausgebildet ist, welche dafür angepasst ist, das medizinische Instrument mit dem Transponder in einer vorbestimmten Position und/oder Ausrichtung relativ zu der Lese- und/oder Schreibvorrichtung zu halten oder temporär zu fixieren, in der eine Signalübertragung zwischen dem Transponder und der Lese- und/oder Schreibvorrichtung ermöglicht ist. In anderen Worten ausgedrückt, kann das medizinische Instrument bzw. die medizinische Vorrichtung von einer Lese- und/oder Schreibvorrichtung ausgelesen und/oder beschrieben werden, die in unmittelbare Nähe, also insbesondere einem Abstand kleiner als ein Zentimeter, zum Transponder gebracht werden kann. Bei medizinischen Instrumenten, die einen Anschluss, zum Beispiel für eine Luftversorgung, eine Stromversorgung und/oder einen Datenaustausch aufweisen, kann diese Auslesevorrichtung in dem koppelbaren Adapter für das Gegenstück an der medizinischen Vorrichtung angebracht sein.

Die Aufgabe der vorliegenden Offenbarung wird hinsichtlich eines Herstellungsverfahrens für ein elektrochirurgisches Instrument, insbesondere für ein Instrument gemäß der vorliegenden Offenbarung, durch die Schritte gelöst: Herstellen, insbesondere werkzeugfallendes Kunststoffspritzgießen, eines Isolationskörpers mit zwei Durchgangsöffnungen und einer Aufnahme für einen Transponder; Einfügen oder Bereitstellen einer Blendenöffnung in einer ersten und/oder zweiten Elektrode; Einsetzen der ersten Elektrode in die erste Durchgangsöffnung und der zweiten Elektrode in die zweite Durchgangsöffnung, Einsetzen des Transponders in die Aufnahme, und vorzugsweise Verschließen der Aufnahme, um den Transponder verliersicher, insbesondere hermetisch einzuschließen.

Gemäß einer Variante kann das Verschließen der Aufnahme durch Kleben, Vergießen durchgeführt werden, oder der Isolationskörper mit einem Hauptkörper mit der Aufnahme mittels eines separaten Verschlusskörper verschlossen wird und dieser Verschlusskörper insbesondere stoffschlüssig mittels Warmverformen oder Ultraschallschweißen mit dem Hauptkörper fest verbunden wird.

### Kurzbeschreibung der Figuren

Die vorliegende Offenbarung wird nachfolgend anhand bevorzugter Ausführungsformen unter Bezugnahme auf die begleitenden Figuren erläutert. Es zeigen:
Fig. 1 eine perspektivische Ansicht eines elektrochirurgischen HF-Instruments gemäß einer ersten bevorzugten Ausführungsform der vorliegenden Offenbarung;
Fig. 2 eine perspektivische Längsschnittansicht eines elektrochirurgischen HF-Instruments gemäß einer weiteren, zweiten bevorzugten Ausführungsform der vorliegenden Offenbarung;
Fign. 3 und 4 jeweils eine weitere perspektivische Ansicht des Instruments aus Fig. 2;
Fig. 5 eine Frontansicht eines Längsschnittes des Instruments aus Fign. 2 bis 4;
Fig. 6 eine perspektivische und teildurchsichtige Ansicht eines elektrochirurgischen HF-Instruments gemäß einer weiteren, dritten bevorzugten Ausführungsform der vorliegenden Offenbarung, bei der die Aufnahme mit einem Pfropfen verschlossen wird;
Fign. 7 bis 11 verschiedene Schnittansichten eines elektrochirurgischen HF-Instruments gemäß einer weiteren, vierten bevorzugten Ausführungsform der vorliegenden Offenbarung, bei der ein Hauptkörper mit einem Verschlusskörper verbunden ist;
Fig. 12 bis 16 verschiedene Schnittansichten eines elektrochirurgischen HF-Instruments gemäß einer weiteren, fünften bevorzugten Ausführungsform der vorliegenden Offenbarung;

Die Figuren sind schematischer Natur und sollen nur dem Verständnis der Erfindung dienen. Gleiche Elemente sind mit denselben Bezugszeichen versehen. Die Merkmale der verschiedenen Ausführungsbeispiele können untereinander ausgetauscht werden.

### Detaillierte Beschreibung bevorzugter Ausführungsformen

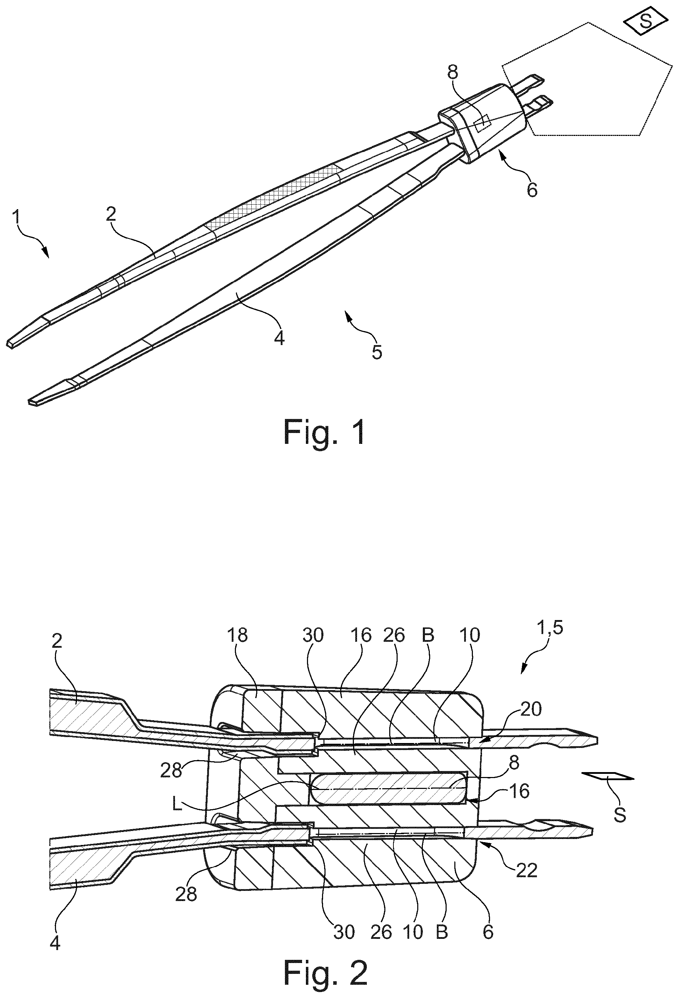

Fig. 1 zeigt ein elektrochirurgisches Instruments 1 gemäß einer ersten bevorzugten Ausführungsform der vorliegenden Offenbarung.

Das elektrochirurgisches Instrument 1 (nachstehend nur als Instrument bezeichnet) ist in Form eines bipolaren HF-Instruments gestaltet und weist eine erste Elektrode 2 und einer zweiten Elektrode 4, die sich distal des Instruments gegenüberliegen. Ferner weist das Instrument 1 einen Isolationskörper 6 auf, in welchem ein Transponder 8 in Form eines Glass-Transponders, aufgenommen ist. Der Isolationskörper 6 zusammen mit den Elektroden 2, 4 und dem Transponder 8 bilden eine Elektrodenvorrichtung 5 als Baugruppe für das Instrument 1. Diese Elektrodenvorrichtung ist an das Instrument an- und abkoppelbar.

Der Isolationskörper verbindet dabei die erste Elektrode 2 und zweite Elektrode 4 miteinander und bildet damit eine Art geometrisches Gehäuse für die beiden Elektroden 2 4 und den Transponder 8. Konkret ist der in dem Isolationskörper 6 aufgenommene Transponder 8 strukturell bzw. geometrisch gesehen symmetrisch und mittig zwischen der ersten Elektrode 2 und zweiten Elektrode 4 angeordnet. Man kann auch sagen, dass auf einer Geraden quer zu einer Längsachse des Isolationskörpers 6 die erste Elektrode 2, der Transponder 8 und die zweite Elektrode 4 in dieser Reihenfolge aufgereiht sind und zwar symmetrisch zueinander.

Ganz speziell ist in dieser Ausführungsform sowohl in der ersten Elektrode 2 als auch in der zweiten Elektrode 4 im Bereich des Transponders 8 bzw. auf Höhe des Transponders 8 eine Blendenöffnung 10 in Form eines Langlochs ausgebildet ist, sodass die Elektroden 2, 4 eine für elektromagnetische Wellen signalundurchlässige Blende 12 mit einer definierten signaldurchlässigen Blendenöffnung 10 ausbildet.

Auf diese Weise lässt sich der Transponder um 360° um das Instrument 1 bzw. die Elektrodenvorrichtung 5 herum auslesen.

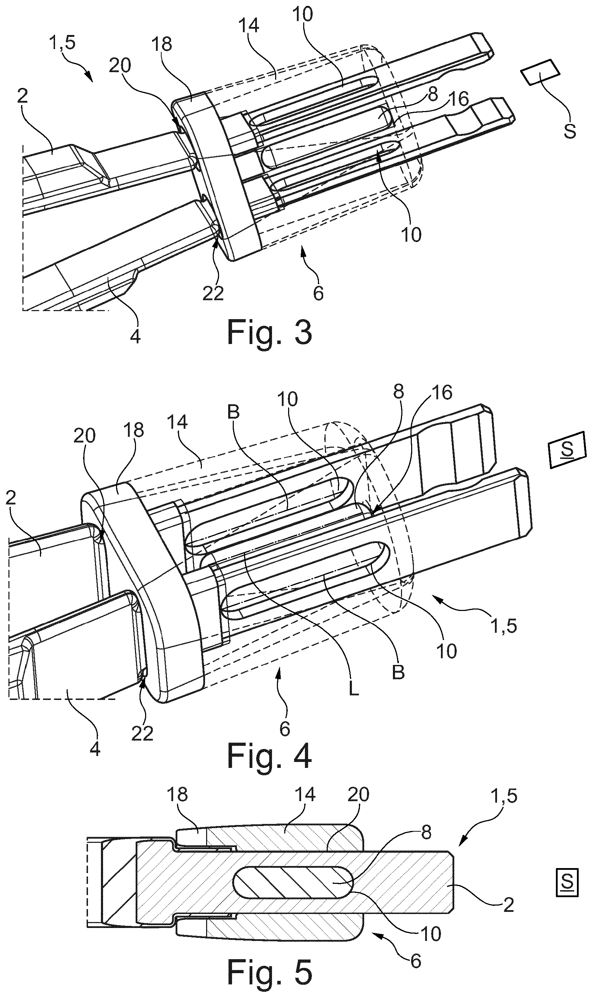

Das HF-Instrument 1 weist also an seiner Elektrodenvorrichtung 5 einen (auf einer Längsachse des Instruments gesehen) proximalen Isolationskörper 6 auf, der elektrisch isolierend ist. Dieser Isolationskörper 6 ist in dieser Ausführungsform einteilig ausgeführt und hat zwei Durchgangsöffnungen 20, 22 in Form von Schlitzen 24 für die Aufnahme bzw. das Einstecken der ersten und zweiten Elektrode 2, 4. Die Aufnahme und feste Verbindung der Elektroden 2, 4 mit dem Isolationskörper 6 erfolgt mittels einer Presspassung (für eine detailliertere Ansicht der einzelnen Merkmale wird auch auf die Fign. 2 bis 5 verwiesen, welche sich ebenfalls Großteils in der Ausführungsform der Fig. 1 wiederfinden).

Der Isolationskörper 6 (also auch die Elektrodenvorrichtung 5) ist symmetrisch zu einer Symmetrieebene S gestaltet. Im Bereich der beiden sich gegenüberliegenden Blendenöffnungen 10, welche in Verlängerung mittig zwischen sich den Transponder 8 vorsehen, ist eine komplementär zum Langloch ausgebildeter Rast-Vorsprung 26 ausgebildet, der formschlüssig in die zugehörige Blendenöffnung 10 eingreift. Da der Isolationskörper 6 als (teil) elastischer Kunststoff (Spritzgussteil) hergestellt ist, kann sich der Rast-Vorsprung 26 auch senkrecht zu einer Längsachse der Elektrodenvorrichtung bzw. senkrecht zur Symmetrieebene (leicht) elastisch verformen. Die Elektroden können also von distal nach proximal in den sich im zusammengebauten Zustand (also mit eingesetztem Transponder 8 und mit Verschließen der Aufnahme) befindlichen Isolationskörper in die Durchgangsöffnungen eingeschoben werden und verrasten elastisch, wenn der Rast-Vorsprung 26 sich in die Blendenöffnung 10 einfügt.

Fig. 2 bis 5 zeigen verschiedene Ansichten einer weiteren Ausführungsform eines elektrochirurgischen Instruments 1 der vorliegenden Offenbarung.

Der Transponder 8 in Form des Glass-Transponders (Glass-Tag als RFID-Transponder) ist wieder mittig im proximalen Isolationskörper 6 in der dafür vorgesehenen Aussparung (welche die Aufnahme 16 bildet) untergebracht bzw. angeordnet. Durch diese Anordnung liegt der RFID (Glass Tag) Transponder 8 wieder genau zwischen den Elektroden 2, 4, die proximal als Anschlusskontakte über den Isolationskörper 6 hinausragen. Durch eine entsprechende geometrische Aussparung der gegenüberliegenden Elektroden im Bereich des Transponders 8 und innerhalb des proximalen Isolationskörpers 6 kann der Transponder 8 in alle Richtungen bzw.um 360° ausgelesen werden. Durch die geometrische Auslegung über den Abstand der Elektroden2, 4, sowie durch die mögliche Definition der Blendenöffnungen 10 bzw. Aussparungen an den Elektroden 2, 4 als Langlöcher mit definierter Länge und Breite kann auch auf die Signalstarke bzw. die Distanz zum Lese- und Schreibgerat Einfluss genommen werden. Besonders bei Anwendung einer NFC Technologie als Transponder und Lese-/Schreibgerät kann dadurch die sehr geringe Lese und Schreibdistanz zum Lese- und Schreibgerat maßgeblich erhöht werden.

In dieser Ausführungsform der Fign. 2 bis 5 ist der Isolationskörper 6 für eine sichere Unterbringung des Transponders 8 in Form des Glass-Tags zweiteilig ausgeführt. Der Isolationskörper 6 weist dabei einen Hauptkörper 14 mit einer Aufnahmeaussparung (in dieser Ausführungsform als einfache Bohrung) als Aufnahme 16 auf und ein zweiter Verschlusskörper 18 als Art komplementärer Deckel fungierend auf. Da der Isolationskörper 6 in dieser Ausführungsform als werkzeugfallendes Kunststoffspritzgussteil ausgeführt ist, kann die Aufnahme(-aussparung) 16 für den Transponder 8 direkt vorgesehen werden.

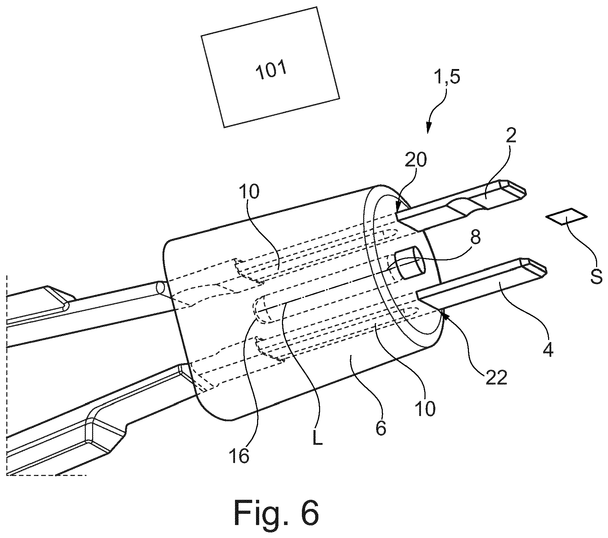

Der Hauptkörper 14 ist mit dem Verschlusskörper 18 über sowohl einen Formschluss als auch über einen elastischen Kraftschluss (elastisch ausgebildeter Isolationskörper 6) gekoppelt. Zudem sind in dem Verschlusskörper 18 ebenfalls weiterführende Durchgangsöffnungen 20, 22 ausgebildet, welche jeweils die Elektroden 2, 4 umfassen und auch hierüber einen Kraftschluss realisieren (ähnlich wie bei einem einteiligen Isolationskörper, wie in nachstehender Fig. 6 gezeigt). Der Zusammenbau von Verschlusskörper 18 (als Art Deckel) und Hauptkörper 14 können also über Form- und/oder Kraftschluss, wie auch bei einem einteiligen Isolationskörper 6 über die Elektroden 2, 4 erfolgen.

Der pillenförmige Transponder 8 liegt wieder, wenn in einer Richtung senkrecht auf die Symmetrieebene S gesehen in Flucht mittig zwischen den beiden konzentrisch zueinander angeordneten Blendenöffnungen 10, so dass auch eine gute Auslesemodalität senkrecht zu der Symmetrieebene S gegeben ist. Konkret ist das Langloch (Blendenöffnung 10) mit gleichen Abmessungen von Länge und Breite ausgebildet wie der pillenförmige Transponder 8. Dadurch ist der Transponder 8 symmetrisch und in Verlängerung zu den Blendenöffnungen 10 sowie auch zurückversetzt angeordnet, und es kann eine Bündelung der elektromagnetischen Strahlung erfolgen mit entsprechender Verbesserung einer datentechnischen Verbindung.

In dieser Ausführungsform ist der Verschlusskörper 18 als eine Art Platte mit zwei senkrecht auf die Platte stehenden Schlitzen 24 gestaltet, wobei die Schlitze 24 nach distal, also in die Richtung von der aus die Elektroden 2, 4 eingeführt werden, einen trichterförmigen Einlauf 28 aufweisen, um noch besser eingeführt zu werden. Mittig zwischen den beiden parallel verlaufenden Schlitzen 24 weist die Platte einen zylinderförmigen Sockel auf, der den gleichen oder leicht größeren Durchmesser (Presspassung) wie die zylinderförmige Aufnahme 16 des Hauptkörpers 14 aufweist. Auf diese Weise wird der Verschlusskörper 18, wenn auf den Hauptkörper aufgesetzt 18 kraftschlüssig (über die Reibung bzw. Presspassung) gehalten und die Aufnahme 16 gegenüber einer Umgebung hin abgedichtet. So kann der Isolationskörper mit dem Transponder 8 etwa sterilisiert werden.

**In** dieser Ausführungsform existiert neben der Symmetrieebene S sogar noch eine weitere zweite Symmetrieebene (senkrecht auf die Symmetrieebene S) in Längsschnittrichtung, wie etwa in Fig. 2 gezeigt.

Der Hauptkörper 14 weist in distaler Richtung zudem eine Stufe 30 auf, als Art Anschlag, sodass die Elektrode 2, 4 nur bis zu diesem Anschlag einsetzbar ist und geometrisch bedingt kein weiteres Einschieben erlaubt (die Elektrode kann nicht versehentlich übermäßig in den Isolationskörper 6 eingeschoben werden. Diese Konfiguration mit Anschlag 30 ist auch in der nachstehend erläuterten Ausführungsform vorgesehen.

Figur 6 zeigt eine weitere Ausführungsform eines elektrochirurgischen Instruments 1, welche sich zu der vorstehenden Ausführungsform im Wesentlichen nur dadurch unterscheidet, dass der Isolationskörper 6 einteilig ausgeführt ist und über einen Kleber verschlossen wird (im Gegensatz zu der zweiteiligen Ausführungsform und nicht über einen Verschlusskörper). Der Isolationskörper 6 weist mittig wieder eine Aussparung als Aufnahme 16 für den Transponder 8 auf, welcher von proximaler Seite her eingesetzt ist. Die Aufnahme 16 mit dem eingesetzten Transponder 8 ist schließlich mittels einer Klebemasse oder Vergussmasse direkt hermetisch dichtend (fluiddicht) verschlossen. Alternativ kann auch eine Art Propfen (als Verschlusskörper, hier nicht dargestellt) eingesetzt werden und dieser über Warmverformen oder Ultraschallschweißen mit dem Isolationskörper (als Hauptkörper) verbunden werden.

Über ein schematisch gezeigtes Transponderkommunikationssystem 101 einer bevorzugten Ausführungsform können Daten des Transponders 8 ausgelesen und auch beschrieben werden. Das Transponderkommunikationssystem weist ein mit dem Transponder 8 des medizinischen Instruments 1 signaltechnisch koppelbaren Lese- und/oder Schreibvorrichtung auf (hier nicht dargestellt), die insbesondere mit einer oder selbst als eine Instrumentenhalterung ausgebildet ist, und welche dafür angepasst ist, das medizinische Instrument 1 mit dem Transponder in einer vorbestimmten Position und/oder Ausrichtung relativ zu der Lese- und/oder Schreibvorrichtung zu halten oder temporär zu fixieren, in der eine Signalübertragung zwischen dem Transponder 8 und der Lese- und/oder Schreibvorrichtung ermöglicht ist.

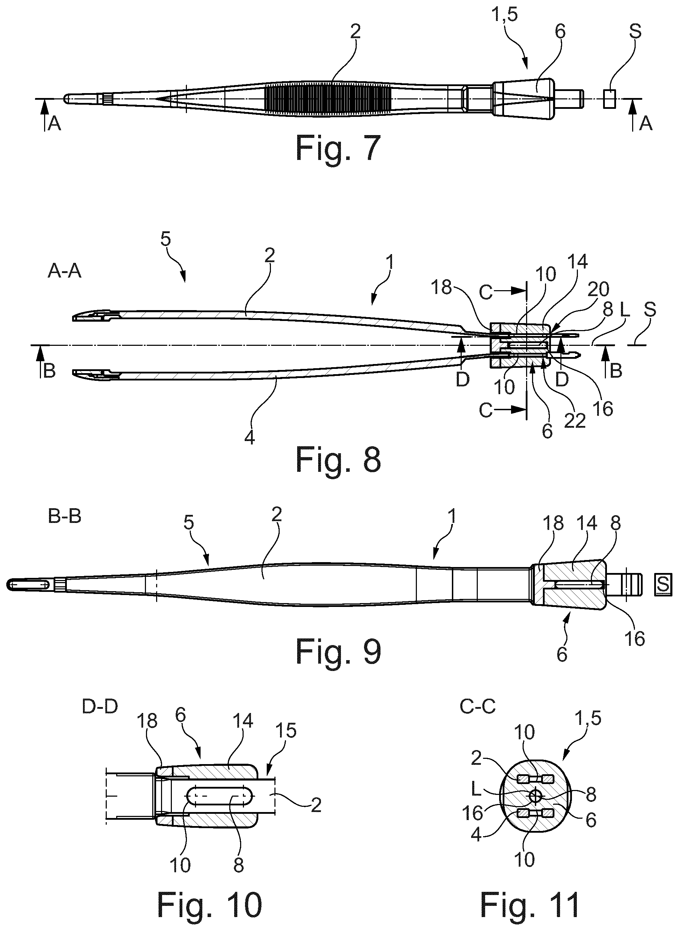

Fign. 7 bis 11 zeigen verschiedene Ansichten einer weiteren Ausführungsform des medizinischen Instruments 1, welches einen zweiteiligen Isolationskörper 6 aufweist, in welchem der Transponder 8 aufgenommen ist.

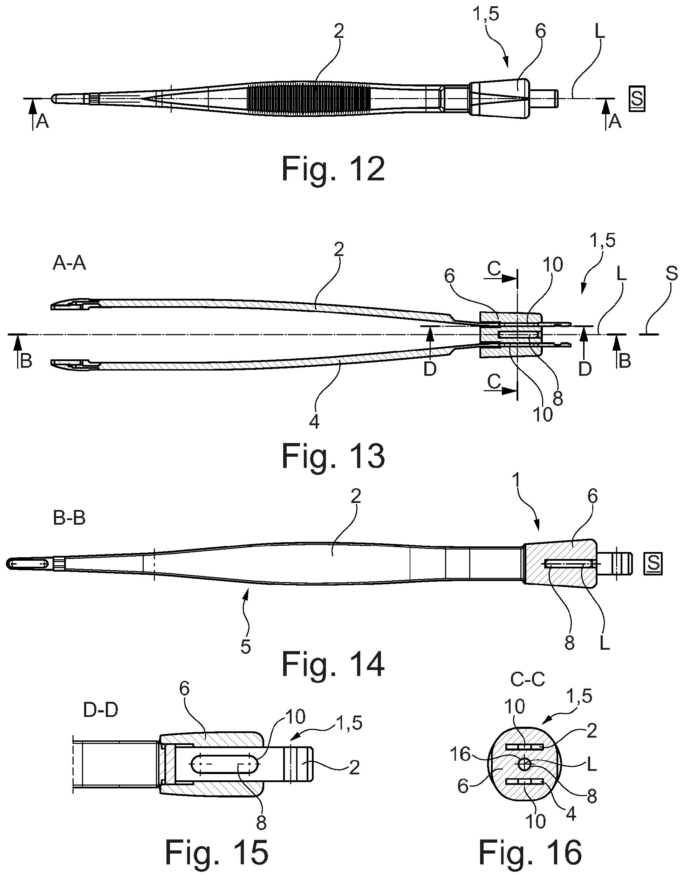

Fign. 12 bis 16 zeigen eine weitere Ausführungsform des elektrochirurgischen Instruments 1 gemäß der vorliegenden Offenbarung. **In** dieser Ausführungsform ist der Isolationskörper wieder einteilig ausgebildet und wird stirnseitig verklebt, sodass der Transponder 8 hermetisch eingeschlossen und fluiddicht in dem Isolationskörper 6 gegenüber der Umgebung versiegelt ist.

Gemäß einem bevorzugten Herstellungsverfahren für ein elektrochirurgisches Instrument gemäß der vorliegenden Offenbarung wird das Instrument hergestellt und montiert mittels der folgenden Schritte.

Bereitstellen S1, insbesondere Herstellen und Bereitstellen, durch werkzeugfallendes Kunststoffspritzgießen eines zweiteiligen Isolationskörpers 6 mit zwei Durchgangsöffnungen 20, 22 für Elektroden 2, 4 und einer Aufnahme 16 für einen Transponder 8.

Hiernach erfolgt der Schritt Einfügen/ Erstellen oder Bereitstellen S2 einer Blendenöffnung 10 in der ersten Elektrode 2 und der zweiten Elektrode 4 des Instruments 1.

In einem nächsten Schritt erfolgt das Einsetzen S3 der ersten Elektrode 2 in die erste Durchgangsöffnung 20 und der zweiten Elektrode 4 in die zweite Durchgangsöffnung 22.

Schließlich wird im Schritt Einsetzen S4 der Transponder 8 in die Aufnahme 16 eingesetzt, und die Aufnahme 16 mittels einer Klebmasse oder mittels eines Verschlusskörpers verschlossen, um den Transponder 8 verliersicher aufzunehmen, in seiner Position zu fixieren, insbesondere in seiner Lage gegenüber den beiden Blendenöffnungen 10 der Elektroden 2, 4, und hermetisch einzuschließen, um eine Sterilität zu gewährleisten.

### Bezugszeichenliste

- 1: Elektrochirurgisches Instrument
- 2: Erste Elektrode
- 4: Zweite Elektrode
- 5: Elektrodenvorrichtung
- 6: Isolationskörper
- 8: Transponder / Glass-Tag
- 10: Blendenöffnung
- 12: Blende
- 14: Hauptkörper
- 16: Aufnahme
- 18: Verschlusskörper
- 20: Erste Durchgangsöffnung
- 22: Zweite Durchgangsöffnung
- 24: Schlitz
- 26: Rast-Vorsprung
- 28: trichterförmiger Einlauf
- 30: Stufe

- 101: Transponderkommunikationssystem

- S: Symmetrieebene
- L: Längsachse
- B: Längsachse Blendenöffnung

- S1: Schritt Herstellen Isolationskörper
- S2: Einfügen Blendenöffnung in Elektrode
- S3: Einsetzen Elektrode in Durchgangsöffnung
- S4: Einsetzen Transponder

## Patentansprüche

1. Elektrochirurgisches Instrument (1), insbesondere HF-Instrument, mit einer Elektrodenvorrichtung (5) mit einer ersten Elektrode (2) und einer zweiten Elektrode (4), die sich, insbesondere distal, gegenüberliegen,
**gekennzeichnet durch**
einen Isolationskörper (6), in welchem ein Transponder (8), bevorzugt ein RFID-Transponder, besonders bevorzugt ein Glass-Transponder, aufgenommen ist, und welcher die erste Elektrode (2) und zweite Elektrode (4) miteinander verbindet,
wobei der in dem Isolationskörper (6) aufgenommene Transponder (8) zwischen der ersten Elektrode (2) und zweiten Elektrode (4) angeordnet ist, insbesondere symmetrisch und/oder mittig zwischen der ersten und zweiten Elektrode (2, 4), und
wobei in der ersten Elektrode (2) und/oder in der zweiten Elektrode (4) im Bereich des Transponders (8) eine Blendenöffnung (10) ausgebildet ist, so dass die Elektrode (2; 4) eine für elektromagnetische Wellen signalundurchlässige Blende (12) mit einer signaldurchlässigen Blendenöffnung (10) ausbildet.

2. Elektrochirurgisches Instrument (1) nach Anspruch 1, **dadurch gekennzeichnet, dass** die Blendenöffnung (10) der Elektrode (2; 4) länglich oder schlitzförmig oder langlochförmig, insbesondere als Langloch, ausgebildet ist.

3. Elektrochirurgisches Instrument (1) nach Anspruch 1 oder 2, **dadurch gekennzeichnet, dass** der Isolationskörper (6) als Material einen thermoplastischen Kunststoff, insbesondere Polypropylen (PP), aufweist, insbesondere aus diesem besteht, vorzugsweise als ein Kunststoffspritzgussteil ausgebildet ist, um eine elektrische Isolation bereitzustellen.

4. Elektrochirurgisches Instrument (1) nach einem der vorangegangenen Ansprüche, **dadurch gekennzeichnet, dass** der Isolationskörper (6) zumindest zweiteilig ausgebildet ist und einen Hauptkörper (14) mit einer Aufnahme (16), insbesondere einer Aussparung, für eine Aufnahme des Transponders (8), und ferner einen Verschlusskörper (18) aufweist, der über einen Form- und/oder Kraftschluss mit dem Hauptkörper (14) verbindbar ist und die Aufnahme (16) hermetisch abschließt, so dass der Transponder (8) verliersicher aufgenommen ist.

5. Elektrochirurgisches Instrument (1) nach Anspruch 4, **dadurch gekennzeichnet, dass** der Verschlusskörper (18) stoffschlüssig mittels Warmverformen oder Ultraschallschweißen mit dem Hauptkörper (14) verbunden ist, um eine permanente Fixierung und hermetische Versiegelung bereitzustellen.

6. Elektrochirurgisches Instrument (1) nach einem der vorangegangenen Ansprüche, **dadurch gekennzeichnet, dass** der Isolationskörper (6) zwei symmetrisch zu einer Längsachse des Isolationskörper verlaufende, insbesondere parallel verlaufende, Durchgangsöffnungen (20, 22), insbesondere Schlitze (24) aufweist, in welche jeweils die erste Elektrode (2) und die zweite Elektrode (4) eingesetzt ist und die jeweils distal und proximal aus dem Isolationskörper (6) vorstehen und insbesondere über eine Presspassung in dem Isolationskörper (6) gehalten werden.

7. Elektrochirurgisches Instrument (1) nach Anspruch 6, **dadurch gekennzeichnet, dass** der Isolationskörper (6) in der Durchgangsöffnung (20; 22), insbesondere dem Schlitz (24), eine Verjüngung oder einen Rast-Vorsprung (26), insbesondere einen komplementär zur Blendenöffnung (10) ausgebildeten Rast-Vorsprung (26), aufweist, so dass das die zugehörige Elektrode (2; 4) mit der Blendenöffnung (10) mittels elastischem Formschluss über den Rast-Vorsprung (26) gehalten ist.

8. Elektrochirurgisches Instrument (1) nach einem der vorangegangenen Ansprüche, **dadurch gekennzeichnet, dass** der Isolationskörper (6), insbesondere der Isolationskörper (6) und die erste und zweite Elektrode (2, 4), symmetrisch zu einer Symmetrieebene (S) ausgebildet ist, so dass auch der Transponder (8) symmetrisch zwischen der ersten und zweiten Elektrode (2, 4) angeordnet ist.

9. Elektrochirurgisches Instrument (1) nach einem der vorangegangenen Ansprüche, **dadurch gekennzeichnet, dass** der Transponder (8) eine zylindrische Form, insbesondere mit abgerundeten Enden, mit einer Transponder-Längsachse (L) aufweist,
die Blendenöffnung (10) der Elektrode (2; 4) länglich oder schlitzförmig oder langlochförmig ausgebildet ist und eine Blendenöffnungs-Längsachse (B) aufweist, und
die Transponder-Längsachse (L) parallel zu der Blendenöffnungs-Längsachse (B), von dieser beabstandet und insbesondere symmetrisch zu dieser angeordnet ist.

10. Elektrochirurgisches Instrument (1) nach einem der vorangegangenen Ansprüche, **dadurch gekennzeichnet, dass**
ein Abstand zwischen dem Transponder (8) und der Blendenöffnung (10) minimal 2mm und/oder maximal 20mm beträgt, insbesondere zwischen einer Transponder-Längsachse (L) und einer Blendenöffnungs-Längsachse (B); und/oder
ein kürzester Abstand zwischen der ersten Elektrode (2) und zweiten Elektrode (4) minimal 4mm und/oder maximal 40mm betragen.

11. Elektrochirurgisches Instrument (1) nach einem der vorangegangenen Ansprüche, **dadurch gekennzeichnet, dass** in dem Isolationskörper (6) eine zylinderförmige Aufnahme in Form einer Aussparung ausgebildet ist, welche in Richtung proximal und/oder distal eine Öffnung aufweist, um den Transponder von proximal oder von distal einzusetzen.

12. Medizinisches Transponderkommunikationssystem (101) **gekennzeichnet durch** ein medizinisches Instrument (1) nach einem der Ansprüche 1 bis 11 und einer mit dem Transponder (8) des medizinischen Instruments (1) signaltechnisch koppelbaren Lese- und/oder Schreibvorrichtung, die insbesondere mit einer oder selbst als eine Instrumentenhalterung ausgebildet ist, welche dafür angepasst ist, das medizinische Instrument (1) mit dem Transponder in einer vorbestimmten Position und/oder Ausrichtung relativ zu der Lese- und/oder Schreibvorrichtung zu halten oder temporär zu fixieren, in der eine Signalübertragung zwischen dem Transponder (8) und der Lese- und/oder Schreibvorrichtung ermöglicht ist.

13. Herstellungsverfahren für ein elektrochirurgisches Instrument, insbesondere für ein Instrument (1) nach einem der Ansprüche 1 bis 11,
**gekennzeichnet durch** die Schritte:
Herstellen (S1), insbesondere werkzeugfallendes Kunststoffspritzgießen, eines Isolationskörpers (6) mit zwei Durchgangsöffnungen (20, 22) für zumindest eine erste und zweite Elektrode (2, 4) und einer Aufnahme (16) für einen Transponder (8);
Einfügen oder Bereitstellen (S2) einer Blendenöffnung (10) in der ersten Elektrode (2) und/oder in der zweiten Elektrode (4) des Instruments (1);
Einsetzen (S3) der ersten Elektrode (2) in die erste Durchgangsöffnung (20) und der zweiten Elektrode (4) in die zweite Durchgangsöffnung (22),
Einsetzen (S4) des Transponders (8) in die Aufnahme (16), und vorzugsweise Verschließen der Aufnahme (16), um den Transponder (8) verliersicher aufzunehmen, insbesondere hermetisch einzuschließen.

14. Herstellungsverfahren nach Anspruch 13, **dadurch gekennzeichnet, dass** das Verschließen der Aufnahme durch Kleben, Vergießen durchgeführt wird, oder
der Isolationskörper (6) mit einem Hauptkörper (14) mit der Aufnahme (16) mittels eines separaten Verschlusskörpers (18) verschlossen wird und dieser Verschlusskörper (18) insbesondere stoffschlüssig mittels Warmverformen oder Ultraschallschweißen mit dem Hauptkörper (14) fest verbunden wird.

## Claims

1. An electrosurgical instrument (1), in particular an HF instrument, with an electrode device (5) with a first electrode (2) and a second electrode (4), which lie opposite each other, in particular distally,
**characterized by**
an insulation body (6), in which a transponder (8), preferably an RFID transponder, particularly preferably a glass transponder, is housed, and which connects the first electrode (2) and second electrode (4) to each other,
wherein the transponder (8) housed in the insulation body (6) is arranged between the first electrode (2) and the second electrode (4), in particular symmetrically and/or centrally between the first and second electrodes (2, 4), and
wherein a screen opening (10) is configured in the first electrode (2) and/or in the second electrode (4) in the region of the transponder (8), so that the electrode (2; 4) forms a screen (12) impermeable to signals of electromagnetic waves with a screen opening (10) permeable to signals.

2. The electrosurgical instrument (1) according to claim 1, **characterized in that** the screen opening (10) of the electrode (2; 4) is elongate or slit-shaped or slot-shaped, is in particular configured as a slot hole.

3. The electrosurgical instrument (1) according to claim 1 or 2, **characterized in that** the insulation body (6) comprises as material a thermoplastic material, in particular polypropylene (PP), in particular consists of this, preferably is configured as a plastic injection-molded part, in order to provide electrical insulation.

4. The electrosurgical instrument (1) according to one of the preceding claims, **characterized in that** the insulation body (6) is configured in at least two parts and has a main body (14) with a receptacle (16), in particular a recess, for receiving the transponder (8), and furthermore has a closure body (18), which is connectable to the main body (14) via a form-fit and/or force-fit and hermetically seals the receptacle (16), so that the transponder (8) is housed in a loss-proof manner.

5. The electrosurgical instrument (1) according to claim 4, **characterized in that** the closure body (18) is firmly bonded to the main body (14) via hot deformation or ultrasonic welding in order to provide a permanent fixation and hermetic seal.

6. The electrosurgical instrument (1) according to one of the preceding claims, **characterized in that** the insulation body (6) has two passage openings (20, 22), in particular slits (24), which run symmetrically to a longitudinal axis of the insulation body, and in particular run parallel, into which the first electrode (2) and the second electrode (4) are respectively inserted and which respectively protrude distally and proximally from the insulation body (6) and in particular are held in the insulation body (6) via a press fit.

7. The electrosurgical instrument (1) according to claim 6, **characterized in that** the insulation body (6), in particular the slit (24), has a tapering or a latch projection (26) in the passage opening (20; 22), in particular a latch projection (26) configured complementary to the screen opening (10), so that the associated electrode (2; 4) is held with the screen opening (10) via an elastic form-fit via the latch projection (26).

8. The electrosurgical instrument (1) according to one of the preceding claims, **characterized in that** the insulation body (6), in particular the insulation body (6) and the first and second electrodes (2, 4), is configured symmetrically to a plane of symmetry (S), so that the transponder (8) is also arranged symmetrically between the first and second electrodes (2, 4).

9. The electrosurgical instrument (1) according to one of the preceding claims, **characterized in that** the transponder (8) has a cylindrical shape, in particular with rounded ends, with a longitudinal axis (L) of the transponder,
the screen opening (10) of the electrode (2; 4) is configured to be elongated or slit-shaped or slot-shaped and has a longitudinal axis (B) of the screen opening, and
the longitudinal axis (L) of the transponder is parallel to the longitudinal axis (B) of the screen opening, spaced apart from it and in particular arranged symmetrically to it.

10. The electrosurgical instrument (1) according to one of the preceding claims, **characterized in that**
a distance between the transponder (8) and the screen opening (10) is a minimum of 2 mm and/or a maximum of 20 mm, in particular between a longitudinal axis (L) of the transponder and a longitudinal axis (B) of the screen opening; and/or
a shortest distance between the first electrode (2) and the second electrode (4) is a minimum of 4 mm and/or a maximum of 40 mm.

11. The electrosurgical instrument (1) according to one of the preceding claims, **characterized in that** a cylindrical receptacle in the form of a recess is configured in the insulation body (6), which has an opening in the proximal and/or distal direction in order to insert the transponder from the proximal or distal side.

12. A medical transponder communication system (101) **characterized by** a medical instrument (1) according to one of claims 1 to 11 and a reading and/or writing device which is coupleable to the transponder (8) of the medical instrument (1) via signal technology and which is configured in particular with or itself as an instrument holder which is adapted to hold or temporarily fix the medical instrument (1) with the transponder in a predetermined position and/or alignment relative to the reading and/or writing device, in which a signal transmission between the transponder (8) and the reading and/or writing device is enabled.

13. A production method for an electrosurgical instrument, in particular for an instrument (1) according to one of claims 1 to 11, **characterized by** the steps of:
producing (S1), in particular off-tool plastic injection molding, an insulation body (6) with two passage openings (20, 22) for at least a first and a second electrode (2, 4) and a receptacle (16) for a transponder (8);
installing or providing (S2) a screen opening (10) in the first electrode (2) and/or in the second electrode (4) of the instrument (1);
inserting (S3) the first electrode (2) into the first passage opening (20) and the second electrode (4) into the second passage opening (22),
inserting (S4) the transponder (8) into the receptacle (16), and preferably closing the receptacle (16) to receive the transponder (8) in a loss-proof manner, in particular hermetically sealing it.

14. The production method according to claim 13, **characterized in that** the closing of the receptacle is performed by gluing, casting, or
the insulation body (6) is closed with a main body (14) with the receptacle (16) via a separate closure body (18) and this closure body (18) in particular is firmly bonded to the main body (14) via hot deformation or ultrasonic welding.

## Revendications

1. Instrument électrochirurgical (1), en particulier instrument HF, avec un dispositif d'électrodes (5) avec une première électrode (2) et une seconde électrode (4) qui se font face, en particulier de manière distale,
**caractérisé par**
un corps d'isolation (6), dans lequel un transpondeur (8), de préférence un transpondeur RFID, le plus préférentiellement un transpondeur en verre, est reçu et relie l'une à l'autre la première électrode (2) et la seconde électrode (4),
dans lequel le transpondeur (8) reçu dans le corps d'isolation (6) est agencé entre la première électrode (2) et la seconde électrode (4), en particulier de manière symétrique et/ou au milieu entre la première et la seconde électrode (2, 4) et
dans lequel une ouverture de diaphragme (10) est conçue dans la première électrode (2) et/ou dans la seconde électrode (4) dans la zone du transpondeur (8), de sorte que l'électrode (2 ; 4) forme un diaphragme (12) imperméable aux signaux pour des ondes électromagnétiques avec une ouverture de diaphragme (10) perméable aux signaux.

2. Instrument électrochirurgical (1) selon la revendication 1, **caractérisé en ce que** l'ouverture de diaphragme (10) de l'électrode (2 ; 4) est conçue de manière oblongue ou en forme de fente ou de trou oblong, en particulier comme trou oblong.

3. Instrument électrochirurgical (1) selon la revendication 1 ou 2, **caractérisé en ce que** le corps d'isolation (6) présente comme matériau une matière thermoplastique, en particulier du polypropylène (PP), en particulier est composé de celui-ci, de préférence est conçu comme une partie moulée par injection de matière plastique afin de fournir une isolation électrique.

4. Instrument électrochirurgical (1) selon l'une quelconque des revendications précédentes, **caractérisé en ce que** le corps d'isolation (6) est conçu au moins en deux parties et présente un corps principal (14) avec un logement (16), en particulier un évidement, pour loger le transpondeur (8), et de plus un corps de fermeture (18) qui peut être relié au corps principal (14) par le biais d'une liaison par complémentarité de formes et/ou à force, et le logement (16) se ferme de manière hermétique de sorte que le transpondeur (8) soit reçu de manière imperdable.

5. Instrument électrochirurgical (1) selon la revendication 4, **caractérisé en ce que** le corps de fermeture (18) est relié au corps principal (14) par complémentarité de matières au moyen d'une déformation à chaud ou d'un soudage par ultrasons afin de fournir une fixation permanente et un scellage hermétique.

6. Instrument électrochirurgical (1) selon l'une quelconque des revendications précédentes, **caractérisé en ce que** le corps d'isolation (6) présente deux ouvertures traversantes (20, 22), en particulier des fentes (24), s'étendant de manière symétrique à un axe longitudinal du corps d'isolation, s'étendant en particulier parallèlement, dans lesquelles respectivement la première électrode (2) et la seconde électrode (4) sont insérées et qui font saillie respectivement de manière distale et proximale du corps d'isolation (6) et sont maintenues en particulier par le biais d'un ajustement par serrage dans le corps d'isolation (6).

7. Instrument électrochirurgical (1) selon la revendication 6, **caractérisé en ce que** le corps d'isolation (6) présente dans l'ouverture traversante (20 ; 22) en particulier la fente (24), un rétrécissement ou une saillie d'encliquetage (26), en particulier une saillie d'encliquetage (26) conçue de manière complémentaire à l'ouverture de diaphragme (10), de sorte que l'électrode (2 ; 4) afférente soit maintenue avec l'ouverture de diaphragme (10) au moyen de la liaison par complémentarité de formes élastique par le biais de la saillie d'encliquetage (26).

8. Instrument électrochirurgical (1) selon l'une quelconque des revendications précédentes, **caractérisé en ce que** le corps d'isolation (6), en particulier le corps d'isolation (6) et les première et seconde électrodes (2, 4) sont conçus de manière symétrique à un plan de symétrie (S), de sorte que le transpondeur (8) soit aussi agencé de manière symétrique entre les première et seconde électrodes (2, 4).

9. Instrument électrochirurgical (1) selon l'une quelconque des revendications précédentes, **caractérisé en ce que** le transpondeur (8) présente une forme cylindrique, en particulier avec des extrémités arrondies, avec un axe longitudinal de transpondeur (L),
l'ouverture de diaphragme (10) de l'électrode (2 ; 4) est conçue de manière oblongue ou en forme de fente ou de trou oblong et présente un axe longitudinal d'ouverture de diaphragme (B) et
l'axe longitudinal de transpondeur (L) est agencé parallèlement à l'axe longitudinal d'ouverture de diaphragme (B) à distance de celui-ci et en particulier de manière symétrique à celui-ci.

10. Instrument électrochirurgical (1) selon l'une quelconque des revendications précédentes, **caractérisé en ce que**
une distance entre le transpondeur (8) et l'ouverture de diaphragme (10) s'élève au minimum à 2 mm et/ou au maximum à 20 mm, en particulier entre un axe longitudinal de transpondeur (L) et un axe longitudinal d'ouverture de diaphragme (B) ; et/ou
une distance la plus courte entre les première électrode (2) et seconde électrode (4) s'élève au minimum à 4 mm et/ou au maximum à 40 mm.

11. Instrument électrochirurgical (1) selon l'une quelconque des revendications précédentes, **caractérisé en ce qu'**un logement cylindrique est conçu sous la forme d'un évidement dans le corps d'isolation (6), évidement qui présente en direction proximale et/ou distale une ouverture afin d'insérer le transpondeur de la direction proximale ou de la direction distale.

12. Système de communication de transpondeur (101) médical **caractérisé par** un instrument médical (1) selon l'une quelconque des revendications 1 à 11 et un dispositif de lecture et/ou d'écriture pouvant être couplé par des signaux au transpondeur (8) de l'instrument médical (1), dispositif qui est conçu en particulier avec ou comme un support d'instrument qui est adapté afin de maintenir l'instrument médical (1) avec le transpondeur dans une position et/ou orientation prédéterminée par rapport au dispositif de lecture et/ou d'écriture ou de le fixer temporairement, dans lequel une transmission de signal est permise entre le transpondeur (8) et le dispositif de lecture et/ou d'écriture.

13. Procédé de fabrication d'un instrument électrochirurgical, en particulier d'un instrument (1) selon l'une quelconque des revendications 1 à 11, **caractérisé par** les étapes suivantes :
la fabrication (S1), en particulier le moulage par injection de matière plastique issu de l'outil d'un corps d'isolation (6) avec deux ouvertures traversantes (20, 22) pour au moins une première et une seconde électrode (2, 4) et un logement (16) pour un transpondeur (8) ;
l'introduction ou la fourniture (S2) d'une ouverture de diaphragme (10) dans la première électrode (2) et/ou dans la seconde électrode (4) de l'instrument (1) ;
l'insertion (S3) de la première électrode (2) dans la première ouverture traversante (20) et de la seconde électrode (4) dans la seconde ouverture traversante (22),
l'insertion (S4) du transpondeur (8) dans le logement (16), et de préférence la fermeture du logement (16) afin de recevoir le transpondeur (8) de manière imperdable, en particulier de l'enserrer hermétiquement.

14. Procédé de fabrication selon la revendication 13, **caractérisé en ce que** la fermeture du logement est réalisée par collage, coulage ou
le corps d'isolation (6) est fermé avec un corps principal (14) avec le logement (16) au moyen d'un corps de fermeture (18) séparé, et ce corps de fermeture (18) est relié fixement au corps principal (14) en particulier par accouplement de matière au moyen d'une déformation à chaud ou d'un soudage par ultrasons.
